# EUROPÄISCHE PATENTSCHRIFT

(11) **EP 0 839 797 B1**
(45) Veröffentlichungstag und Bekanntmachung des Hinweises auf die Patenterteilung: **31.05.2000**
(21) Anmeldenummer: 97118409.8
(22) Anmeldetag: 23.10.1997
(51) Int. Cl.: C07C 209/62, C07C 211/11, C07D 239/06

(54) **Verfahren zur Herstellung von 2-Methyl-2,4-diaminopentan**
Process for the preparation of 2-methyl-2,4-diaminopentane
Procédé pour la préparation du 2-méthyl-2,4-diaminopentane

(30) Priorität: 05.11.1996 DE 19645549
(43) Veröffentlichungstag der Anmeldung: 06.05.1998
(73) Patentinhaber: BAYER AG, 51368 Leverkusen (DE)
(72) Erfinder: Waldmann, Helmut, Dr., 52385 Nideggen (DE); Dahmer, Jürgen, Dr., 47800 Krefeld (DE); Bazanov, Anatoly, Prof., 193318 St. Petersburg (RU); Timofeev, Alexandre, Dr., 195253 St. Petersburg (RU); Zubritskaya, Natalja, Dr., 190068 St. Petersburg (RU); Terechtchenko, Gennady, Prof., 195220 St. Petersburg (RU)

(56) Entgegenhaltungen:
- DE-B- 1 276 041
- US-A- 2 486 648
- US-A- 3 904 625

## Beschreibung

Die Erfindung betrifft ein Verfahren zur Herstellung von 2-Methyl-2,4-diaminopentan aus 2,2,4,4,6-pentamethyl-2,3,4,5-tetrahydropyrimidin, 2-Methyl-2,4-diaminopentan ist ein wichtiges Zwischenprodukt, z.B. für Lackrohstoffe.

Die Herstellung von 2-Methyl-2,4-diaminopentan durch katalytische Reduzierung von 2,2,4,4,6-Pentamethyl-2,3,4,5-tetrahydropyrimidin mit Wasserstoff/Ammoniak ist bekannt (z.B. US-A 2 486 648). Jedoch ist dieses Verfahren nicht unproblematisch, da dieses Pyrimidinderivat unter Reaktionsbedingungen bei höheren Temperaturen in Gegenwart von Wasser unerwünschte Nebenreaktionen eingeht.

Die Verwendung von Ionenaustauscherharzen als Katalysator zur Herstellung von Pyrimidinen, u.a. auch Acetonin ist in US-A-3 904 625 bekannt. In DE-B-1 276 041 wird ein weiteres Katalysatorsystem zur Gewinnung des Acetonins aus Aceton und Ammoniak gelehrt.

Es war daher Aufgabe der Erfindung ein ergiebigeres Herstellungsverfahren für 2-Methyl-2,4-diaminopentan zu finden.

Es wurde nun gefunden, daß 2-Methyl-2,4-diaminopentan aus Aceton, Ammoniak und Wasserstoff nach dem folgenden Schema hergestellt werden kann:

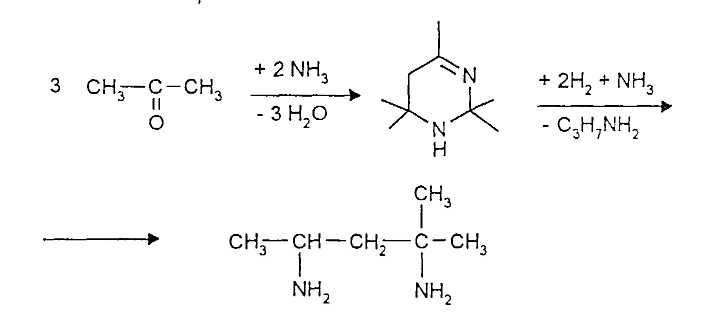

Gegenstand der Erfindung ist ein Verfahren zur Herstellung von 2-Methyl-2,4-diaminopentan, dadurch gekennzeichnet, daß Aceton, Ammoniak und Wasserstoff bei einer Temperatur von 20 bis 130°C und bei Drücken von 30 bis 250 bar durch ein Zwei-Zonenkatalysator geleitet werden, wobei die erste Zone mit einem NH₄^{⊕}-gruppenhaltigen Kationenaustauscherharz oder einem Katalysator gefüllt ist, der aus Aluminiumoxid und/oder Siliziumoxid besteht und anschließend in die zweite Zone aus einen Nickel enthaltenden Hydrierkatalysator besteht.

Erfindungsgemäß können Aceton bzw. Verbindungen eingesetzt werden, die bei den Umsetzungsbedingungen Aceton liefern bzw. aus Aceton gebildet werden, z.B. Diacetonalkohol, Acetonimine wie Acetonimin usw.

Die Umsetzung wird bei einer Temperatur von 20 bis 150°C, bevorzugt 50 bis 130°C durchgeführt, bei einem Druck von 10 bis 300 bar, bevorzugt 30 bis 250 bar.

Das erfindungsgemäße Verfahren wird in zwei Zonen durchgeführt. In der ersten Zone befindet sich als Katalysator ein übliches sulfoniertes Kationenaustauscherharz in der NH₄-Form oder Al- oder Si-Oxide und in der zweiten Zone ein Katalysator, der 30 - 60 Gew.-% Nickel und/oder Kupfer auf Chromoxid enthält.

Bei der Durchführung des Verfahrens werden die Ausgangsmaterialien Aceton bzw. Substanzen die Aceton unter den Umsetzungsbedingungen liefern, Ammoniak und Wasserstoff in eine erste Reaktionszone gebracht, die als Katalysator sulfoniertes Polystyrolharz in der NH₄-Form enthält und anschließend in eine zweite Zone geleitet, die als Katalysator, Nickel auf Chromoxid enthält, der vor Beginn der Umsetzung mit Wasserstoff beladen wurde.

Nach Durchlaufen der Reaktionszonen wird das erhaltene Produkt durch Destillation aufgearbeitet.

### Beispiele

### Beispiel 1

Zur Durchführung des Verfahrens werden zwei Reaktoren mit Festbettkatalysatoren, welche hintereinandergeschaltet sind, verwendet. Der erste Reaktor wurde mit 200 cm² eines sulfonierten Polystyrolkationaustauscherharzes in der NH₄-Form (Ku-2-8) beschickt und der zweite Reaktor mit 500 cm² eines reduzierten Katalysators der 48 Gew.-% Nickel auf Chromoxid enthielt. Aceton wurde mit einer Durchflußrate von 50 ml/h und flüssiger Ammoniak mit einer Durchflußrate von 20 ml/h von unten in den ersten Reaktor eingespeist, wobei die Temperatur bei 20 bis 30°C (Raumtemperatur) gehalten wurde, und der Druck ca. 50 bar betrug. Nachdem die Reaktionsmischung den ersten Reaktor passiert hatte, wurde auf 120°C erhitzt und in den zweiten Reaktor zusammen mit flüssigem Ammoniak (150 ml/h) und Wasserstoff (200 l/h) geleitet. Im zweiten Reaktor betrug die Temperatur 120°C bei einem Druck von 200 bar.

Nach Passieren des zweiten Reaktors wurde überschüssiges Ammoniak aus der Reaktionsmischung abdestilliert. Pro Stunde wurden 41 g eines Produktes erhalten, welches 33,5 Gew.-% 2-Methyl-2,4-diaminopentan enthielt (Ausbeute 65 % der Theorie).

### Beispiel 2

Das Verfahren wurde wie in Beispiel 1 durchgeführt. Der erste Reaktor wurde mit 200 cm² eines Katalysators beschickt, der 17 Gew.-% Al.₂O₃ auf Silicagel als Katalysator enthielt. Der zweite Reaktor wurde mit 200 cm² eines Katalysators gefüllt, der 46 Gew.-% Nickel und 17 Gew.-% Kupfer auf Chromoxid enthielt. Der Katalysator im zweiten Reaktor wurde mit Wasserstoff reduziert. Dann wurde Aceton und flüssiger Ammoniak mit einer Rate von jeweils 10 ml/h vom Boden her in den ersten Reaktor eingeleitet, bei einer Temperatur von 90°C und dann in den zweiten Reaktor bei einer Temperatur von 130°C, wobei der Druck in beiden Reaktoren 100 bar betrug.

Nachdem die Reaktionsmischung durch den ersten Reaktor geleitet worden war, wurde sie zusammen mit Ammoniak mit einer Rate von 30 ml/h und Wasserstoff mit einer Rate von 100 l/h in den zweiten Reaktor eingeleitet.

Nach Passieren des zweiten Reaktors und Abdestillieren des Ammoniaks aus der Reaktionsmischung wurde mit einer Rate von 12,2 g/h ein Produkt enthalten, daß 24,8 Gew.-% 2-Methyl-2,4-diaminopentan (48 % der Theorie enthielt).

## Patentansprüche

1. Verfahren zur Herstellung von 2-Methyl-2,4-diaminopentan, dadurch gekennzeichnet, daß Aceton, Ammoniak und Wasserstoff bei einer Temperatur von 20 bis 130°C und bei Drücken von 30 bis 250 bar durch ein Zwei-Zonenkatalysator geleitet werden, wobei die erste Zone mit einem NH₄^{⊕} -gruppenhaltigen Kationenaustauscherharz oder einem Katalysator gefüllt ist, der aus einem Gemisch von Aluminiumoxid und/oder Siliziumoxid besteht, und anschließend in die zweite Zone aus einem Nickel enthaltenden Hydrierkatalysator besteht.

## Claims

1. Process for the production of 2-methyl-2,4-diaminopentane, characterised in that acetone, ammonia and hydrogen are passed through a two-zone catalyst at a temperature of 20 to 130°C and at pressures of 30 to 250 bar, wherein the first zone is filled with a cation exchange resin containing NH₄^{⊕} groups or a catalyst which consists of a mixture of aluminium oxide and/or silicon oxide and then in the second zone consists of a hydrogenation catalyst containing nickel.

## Revendications

1. Procédé de préparation du 2-méthyl-2,4-diaminopentane, caractérisé en ce que l'on envoie de l'acétone, de l'ammoniac et de l'hydrogène à une température de 20 à 130°C sous des pressions de 30 à 250 bar sur un catalyseur à deux zones, la première zone étant garnie d'une résine échangeuse de cations à groupes NH₄^{⊕} ou d'un catalyseur consistant en un mélange d'alumine et/ou de silice, la deuxième zone consistant ensuite en un catalyseur d'hydrogénation qui contient du nickel.
